# EUROPEAN PATENT APPLICATION

(11) **EP 3 660 046 A1**
(43) Date of publication of application: **03.06.2020**
(21) Application number: 18209444.1
(22) Date of filing: 30.11.2018
(51) Int. Cl.: C07K 16/18, C07K 16/22, C07K 16/24, A61P 35/00

(54) **COMBINATION OF INHIBITORS OF IL-6, VEGF-A, AND MFGE-8**

(71) Applicant: Univerzita Karlova, 11636 Praha 1 (CZ); Ustav molekularni genetiky AV CR, v.v.i., 142 20 Praha 4 (CZ); Vychodoslovensky ustav srdcovych a cievnych chorob a.s., 04011 Kosice (SK)
(72) Inventor: Strnad, Hynek, 251 01 Svetice (CZ); Kolá , Michal, 169 00 Praha 6 (CZ); Smetana, Karel, 141 00 Praha 4 (CZ); Dvoránková, Barbora, 130 00 Praha 3 (CZ); Szabo, Pavol, 04012 Kosice (SK); Lacina, Luká, 143 00 Praha 12 (CZ); Novák, Stepán, 150 00 Praha 5 (CZ); Gál, Peter, 040 00 Kosice (SK)
(74) Representative: Hartvichova, Katerina

(57) **Abstract**

The present invention relates to a combination of at least one IL-6 inhibitor, at least one VEGF-A inhibitor, and at least one MFGE-8 inhibitor. The invention further relates to a pharmaceutical preparation containing this combination of inhibitors, and to the use thereof as a medicament.

## Description

### Field of Art

The present invention relates to a combination of inhibitors for use as a medicament, in particular in treatment of cancer, the inhibitors being low-molecular inhibitors and/or antibodies blocking bioactive proteins produced by cancer-associated fibroblasts. The invention further relates to a pharmaceutical preparation containing this combination of inhibitors.

### Background Art

Malignant tumors represent complicated ecosystems formed by cancer cells and elements that form cancer stroma such as cancer-associated fibroblasts (CAFs), leukocytes and blood/lymphatic capillaries (Lacina L, Kodet O, Dvořánková B, Szabo P, Smetana K Jr.: Ecology of melanoma cell. Histol Histopathol 33: 247-254, 2018). All these cells communicate through mutual intercellular contacts, autocrine/paracrine signaling via bioactive factors such as growth factors, cytokines, chemokines and enzymes, as well as by production of extracellular matrix molecules (Gál P, Varinská L, Fáber L, Novak Š, Szabo P, Mitrengová P, Mirossay A, Mučaji P, Smetana K. Jr.: How signaling molecules regulate tumor microenvironment: Parallels to wound repair. Molecules. 22: pii: E1818, 2017; Zivicova, V., Gal, P., Mifkova, A., Novak, S., Kaltner, H., Kolar, M., Strnad, H., Sachova, J., Hradilova, M., Chovanec, M., Gabius, H.-J., Smetana, K. Jr., Fik, Z.: Detection of distinct changes in gene-expression profiles in specimens of tumors and transition zones of tenascin positive/-negative head and neck squamous cell carcinoma. Anticancer Res. 38: 1279-1290, 2018). Bulk of data has demonstrated that the cells and molecules present in malignant tumors form micromilieu that are important for control of biological properties of many tumor types. Genetic and bio-/immunohistochemic analyses have shown that IL-6 and IL-8 represent very important factors of these cancer microenvironments and influence differentiation pattern of cancer cells and their invasive potential (Kolář M, Szabo P, Dvořánková B, Lacina L, Gabius H-J, Strnad H, Šáchová J, Vlček C, Plzák J, Chovanec M, Cada Z, Betka J, Fik Z, Pačes J, Kovářová H, Motlík J, Jarkovská K, Smetana K Jr.: Upregulation of IL-6, IL-8 and CXCL-1 production in dermal fibroblasts by normal/malignant epithelial cells in vitro, immunohistochemical and transcriptomic analyses. Biol Cell 104: 738-751, 2012; Smetana K Jr, Dvořánková B, Lacina L, Strnad, H, Kolář M, Chovanec M, Plzák J, Cada Z, Vlček C, Szabo P, Betka J, Motlík J, Kovářová H, Jarkocvská K: Combination of antibodies or Fab fragments thereof for use as medicament, and pharmaceutical composition containing said antibodies or Fab fragments thereof . Czech National Patent 303 227, 2012; Szabo, P., Valach, J., Smetana, K. Jr., Dvořánková, B.: Comparative Analysis of Production of IL-8 and CXCL-1 by Normal and Cancer Stromal Fibroblasts. Folia Biol. 59: 134-137, 2013; Kodet O., Lacina L, Krejčí E., Dvořánková B., Grim M., Štork J., Kodetová D., Vlček C., Šáchová J., Kolář M., Strnad H., Smetana K. Jr.: Melanoma cells influence the differentiation pattern of human epidermal keratinocytes. Mol. Cancer 14: 1, 2015; Jobe, N., P., Rösel, D., Dvořánková, B., Kodet, O., Lacina, L., Mateu, R., Smetana, K., Jr., Brábek, J: Simultaneous blocking of IL-6 and IL-8 is sufficient to fully inhibit CAF-induced human melanoma cell invasiveness. Histochem Cell Biol. 146: 205-217, 2016; Jayatilaka H, Tyle P, Chen JJ, Kwak M, Ju J, Kim HJ, Lee JSH, Wu PH, Gilkes DM, Fan R, Wirtz D: Synergistic IL-6 and IL-8 paracrine signalling pathway infers a strategy to inhibit tumour cell migration. Nat Commun. 2017 May 26;8:15584; Jobe, N. P., Živicová, V., Mifková, A., Rösel, D., Dvořánková,B., Kodet O., Strnad, H., Kolář, M., Šedo, A., Smetana, K. Jr., Strnadová, K., Brábek, J., Lacina, L.: Fibroblasts potentiate melanoma cells in vitro invasiveness induced by UV-irradiated keratinocytes. Histochem Cell Biol, DOI: HACB-D-18-00001R1, 2018; Kodet O., Dvořánková B., Bendlová B., Sýkorová V., Krajsová I., Štork J, Kučera J., Szabo P., Strnad H., Kolář M., Vlček C., Smetana K. Jr., Lacina L.: Microenvironment-driven resistance to B -Raf inhibition in a melanoma patient is accompanied by broad changes of gene methylation and expression in distal fibroblasts. Int J Mol Med 41: 3687-2703, 2018). Although this evidence on the cancer micromilieux is very intriguing, it is not clear whether there is some universal cancer micromilieu feature independent of cancer type or whether tumors of distinct cancer types require specific micromilieux (Lacina, L. Plzak J., Kodet O., Szabo P., Chovanec, M., Dvorankova B., Smetana, K. Jr.: Cancer microenvironment: What can we learn from the stem cell niche. Int J Mol Sci 16: 24094-24110, 2015). Our previous research has demonstrated that CAFs isolated from squamous cell carcinoma, basal cell carcinoma, skin metastasis of the breast cancer and skin metastasis of malignant melanoma respectively, significantly influence phenotype of breast cancer cells to more aggressive appearance (Dvořánková, B., Szabo, P., Lacina, L., Kodet, O., Matoušková, E., Smetana, K., Jr.: Fibroblasts prepared from different types of malignant tumors stimulate expression of luminal marker keratin 8 in the EM-G3 breast cancer cell line. Histochem. Cell Biol. 137: 679-685, 2012). Similarly, CAFs prepared from malignant melanoma significantly stimulate invasiveness of glioblastoma cells (Trylcova, J., Busek, P., Smetana, K. Jr., Balaziova, E., Dvorankova, B., Mifkova A., Sedo, A.: Effect of cancer-associated fibroblasts on the migration of glioma cells in vitro. Tumor Biol 36: 5873-5879, 2015). These pieces of evidence indicate that CAFs originating from one cancer type can influence cancer cells of another cancer type and thus that the impact of CAFs on cancer cells is independent of tumor type. This observation may have important ramifications for therapeutic manipulation of cancer stroma in the course of anticancer therapy.

### Disclosure of the Invention

The present invention relates to a combination of inhibitors for use as a medicament, wherein the combination contains inhibitors for binding to IL-6, VEGF-A and MFGE-8 proteins or to receptors of these proteins present on cancer-associated fibroblasts (CAFs) producing these proteins or to receptors of these proteins present on target cancer cells, and wherein the inhibitors are low-molecular inhibitors and/or anti- IL-6 and/or anti-VEGF-A and/or anti-MFGE-8 antibodies and/or Fab fragments thereof.

The object of the present invention is a combination of at least one IL-6 inhibitor, at least one VEGF-A inhibitor, and at least one MFGE-8 inhibitor. IL-6 inhibitor is a compound binding to IL-6 protein or to IL-6 receptor (IL-6R). VEGF-A inhibitor is a compound binding to VEGF-A protein or to VEGF-A receptor (VEGF-A R). MFGE-8 inhibitor is a compound binding to MFGE-8 protein or to MFGE-8 receptor (MFGE-8R).

The inhibitors can thus be defined as compounds, which are capable of binding directly towards IL-6, VEGF-A and/or MFGE-8 proteins and/or towards the receptors of IL-6, VEGF-A and/or MFGE-8 proteins (IL-6R, VEGF-AR and/or MFGE-8R) present on cancer-associated fibroblasts (CAFs) producing the IL-6, VEGF-A and/or MFGE-8 proteins or on target cancer cells. Anti- IL-6, anti-VEGF-A and anti-MFGE-8 antibodies are commercially available as VEGF-A antibody (purchased as clone 26503, cat.number:MAB293, RnD System), recombinant VEGF-A (purchased as cat.number: 293-V-050, RnD System), recombinat IL-6 (purchased as cat.number: 206-IL, RnD System), IL6 antibody (purchased as cat.number: AF-206-NA, RnD System), recombinant MFG-E8 (purchased as cat.number:2767-MF-050, RnD System), MFG-E8 antibody (purchased as cat.number: 11-113-C100, Exbio Antibodies).

It is known that cells and molecules present in malignant tumors form micromilieu important for control of biological properties of many tumor types. The inventors have found that addition of the combination of IL-6, VEGF-A and MFGE-8 proteins into normal (healthy) fibroblast culture significantly stimulated coexpression of cancer markers (keratin type 8 and keratin type 14), and have demonstrated that blocking the activity of IL-6, VEGF-A and MFGE-8 proteins significantly, in synergistic manner, reduced number of cancer cells. The simultaneous inhibition of the three above mentioned proteins changes significantly the differentiation of the tumour cells towards nearly normal degree of differentiation (by more than 70 %), whereas inhibition solely of eather one of these proteins decreases the differentiation of the tumour cells by only less than 10 %. The combination according to the present invention is therefore capable of improving the efficiency of traditional terapeutic methods in cancer treatment (surgery, chemotherapy) by influencing significantly the differentiation of the tumour cells.

In one embodiment, the combination according to the present invention contains:
- IL-6 inhibitor, selected from a low-molecular inhibitor of the IL-6 protein, a low-molecular inhibitor of the IL-6 receptor, an anti-IL-6 antibody, an anti-IL-6R antibody, a Fab fragment of anti-IL-6 antibody, a Fab fragment of anti-IL-6R antibody;
- the VEGF-A inhibitor, selected from a low-molecular inhibitor of the VEGF-A protein, a low-molecular inhibitor of the VEGF-A receptor, an anti-VEGF-A antibody, an anti-VEGF-AR antibody, a Fab fragment of anti-VEGF-A antibody, a Fab fragment of anti-VEGF-AR antibody;
- MFGE-8 inhibitor, selected from a low-molecular inhibitor of the MFGE-8 protein, a low-molecular inhibitor of the MFGE-8 receptor, an anti-MFGE-8 antibody, an anti-MFGE-8R antibody, a Fab fragment of anti-MFGE-8 antibody, a Fab fragment of anti-MFGE-8R antibody. Low-molecular inhibitor is defined as a substance (compound) having its molecular weight up to 5000 g/mol, preferably up to 2000 g/mol, more preferably up to 1000 g/mol.

In one preferred embodiment, the combination according to the present invention contains anti- IL-6, anti-VEGF-A and anti-MFGE-8 antibodies and/or Fab fragments thereof.

In one preferred embodiment, the combination according to the present invention further comprises at least one IL-8 inhibitor for binding directly to IL-8 protein or for binding to receptors of IL-8 protein, present on cancer associated fibroblasts (CAFs) producing this protein or on cancer cells. Low-molecular inhibitors of IL-8 are known in the state of the art, for example the low-molecular inhibitor (2R)-2-[4-(2-methylpropyl)phenyl]-N-methylsulfonylpropanamide (C₁₄H₂₁NO₃S, generic name reparixin, produced by Dompe, Milan, Italy). High-molecular inhibitor for binding to IL-8 protein or to IL-8R is preferably selected from anti-IL-8 antibody, anti-IL-8R antibody, a Fab fragment of anti-IL-8 antibody, a Fab fragment of anti-IL-8R antibody. Anti- IL-8 antibody is commercially available as rekombinant IL-8 (purchased as cat.number:208-IL/CF, RnD System) and IL-8 antibody (purchased as clone 6217, cat.number:MAS208, RnD System). The tests performed by the inventors revealed that addition of IL-8 inhibitor to the combination according to the present invention further improved the inhibitory effect of the combination.

In one preferred embodiment, the combination according to the present invention contains the at least one IL-8 inhibitor, which is selected from a low-molecular inhibitor of the IL-8 protein, a low-molecular inhibitor of the IL-8 receptor, an anti-IL-8 antibody, an anti-IL-8R antibody, a Fab fragment of anti-IL-8 antibody, a Fab fragment of anti-IL-8R antibody. More preferably, the at least one IL-8 inhibitor is reparixin.

In one prefered embodiment of the present invention, the combination according to the present invention contains anti- IL-6, anti-VEGF-A and anti-MFGE-8 antibodies or Fab fragments thereof, or anti- IL-6R, anti-VEGF-AR and anti-MFGE-8R antibodies or Fab fragments thereof, which are humanized. Humanization thereof minimizes the risk of alergic reactions of patients as would be in case of animal antibodies.

In one embodiment, the combination for use according to the present invention contains IL-6 inhibitor, VEGF-A inhibitor and MFGE-8 inhibitor in molar ratios IL-6 inhibitor: VEGF-A inhibitor: MFGE-8 inhibitor 1-5 : 1-5 : 1-5.

In one preferred embodiment, the combination for use according to the present invention contains IL-6 inhibitor, VEGF-A inhibitor, MFGE-8 inhibitor and IL-8 inhibitor in molar ratios IL-6 inhibitor: VEGF-A inhibitor: MFGE-8 inhibitor: IL-8 inhibitor 1-5 : 1-5 : 1-5 : 1-5.

The object of the present invention is further a pharmaceutical preparation, which contains the combination of inhibitors according to the present invention as defined above, the combination containing at least one IL-6 inhibitor, at least one VEGF-A inhibitor, and at least one MFGE-8 inhibitor, and at least one pharmaceutically acceptable carrier. The pharmaceutically acceptable carrier is preferably selected from preservatives, stabilisers, wetting agents and/or emulsifiers, solubilizing agents, salts for regulating the osmotic pressure and/or buffers.
Pharmaceutical preparations are suitable for parental administration, and they are aqueous solutions of the combination of inhibitors according to the present invention in water-soluble form or aqueous injection suspensions, which comprise viscosity-increasing substances, for example sodium carboxymethylcellulose, sorbitol and/or dextran, and if appropriate, stabilizers. The combination of inhibitors according to the present invention can also be present in the form of a lyophilisate, if appropriate together with excipients, and be dissolved before parenteral administration by addition of suitable solvents. Preferred preservatives are, for example, antioxidants, such as ascorbic acid, or microbicides, such as sorbic or benzoic acid.

In one prefered embodiment, the pharmaceutical preparation further contains at least one cytostatic.

In one embodiment, the concentrations of anti- IL-6 antibody, anti-IL-6R antibody, the Fab fragment of anti-IL-6 antibody, the Fab fragment of anti-IL-6R antibody, anti-VEGF-A antibody, anti- VEGF-AR antibody, a Fab fragment of anti- VEGF-A antibody, a Fab fragment of anti-VEGF-AR antibody, anti-MFGE-8 antibody, anti- MFGE-8R antibody, a Fab fragment of anti-MFGE-8 antibody, a Fab fragment of anti- MFGE-8R antibody, anti-IL-8 antibody, an anti-IL-8R antibody, a Fab fragment of anti-IL-8 antibody, a Fab fragment of anti-IL-8R antibody in the pharmaceutical preparation according to the present invention contains range from 10 to 50 µg/ml.

In another embodiment, the pharmaceutical preparation according to the present invention comprises IL-6, VEGF-A and MFGE-8 inhibitors, each of them in the concentration ranging from 10 to 50 µg/ml, and it further comprises from 10 to 50 µg/ml of IL-8 inhibitor, preferably of the low-molecular inhibitor with generic name reparixin or of anti-IL-8 antibody or of Fab fragments thereof.

The object of the present invention is further the combination and/or the pharmaceutical preparation according to the present invention for use as a medicament.

The object of the present invention is also the combination and/or the pharmaceutical preparation according to the present invention for use in the treatment of cancer, preferably selected from the group comprising breast cancer, malignant melanoma, head and neck squamous cell carcinoma and other.

### Brief description of Drawings

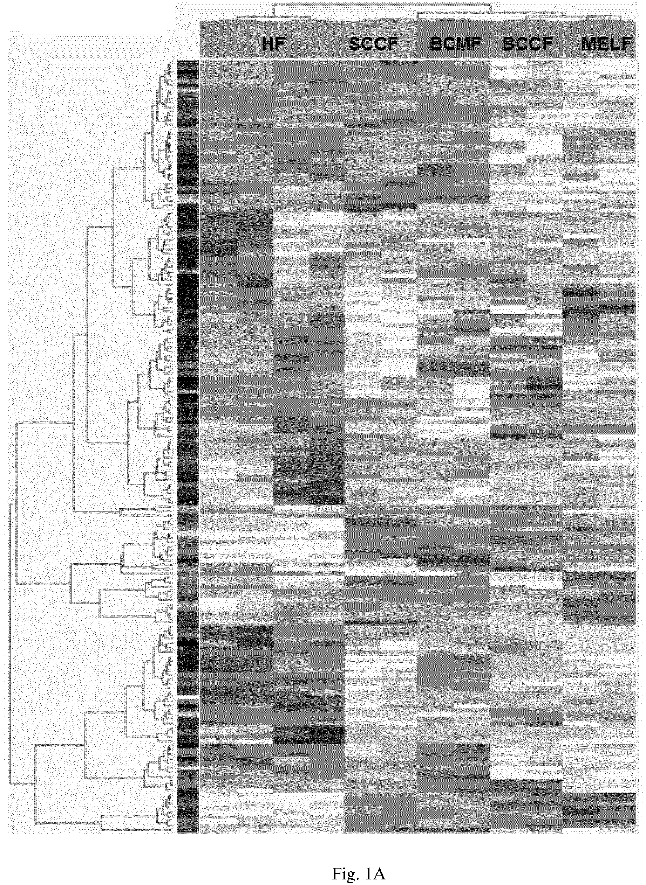

**Fig. 1A****: Heatmap.** Figure demonstrates comparative whole genome analysis of expression profiles of normal (healthy) fibroblasts (HF), cancer-associated fibroblasts (CAFs) from the squamous cell carcinoma of the head and neck (SCCF), basal cell carcinoma (BCCF), skin metastasis of breast cancer (BCMF) and skin metastasis of melanoma (MELF) where expression profile of normal fibroblasts (HF) significantly differs from cancer-associated fibroblasts (BCCF, SCCF, BCMF and MELF).

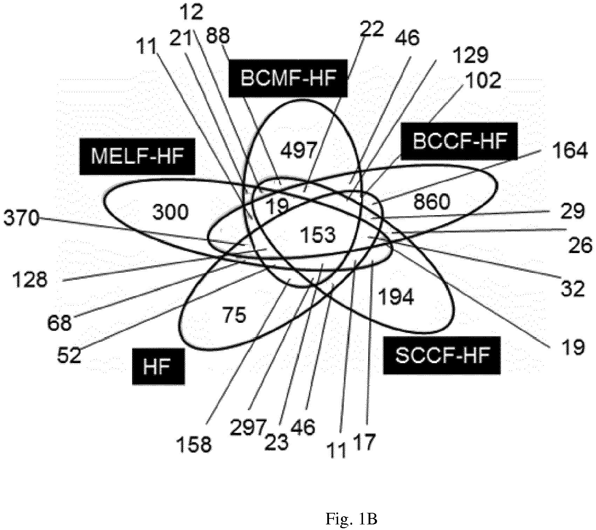

**Fig. 1B****: Venn diagram.** The Venn diagram shows 19 genes, which form the intersection of significantly upregulated genes in all four CAFs but not in the HF.

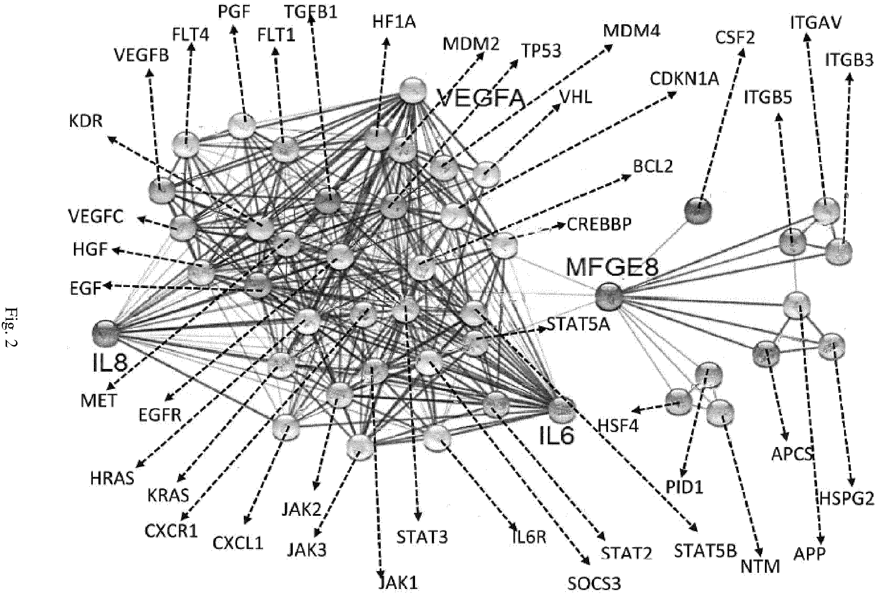

**Fig. 2****: Protein strings** demonstrate relations between IL-6, IL-8, MFGE8 and VEGFA to other proteins important for cancer cell biology according to bioinformatic analysis (Protein String).

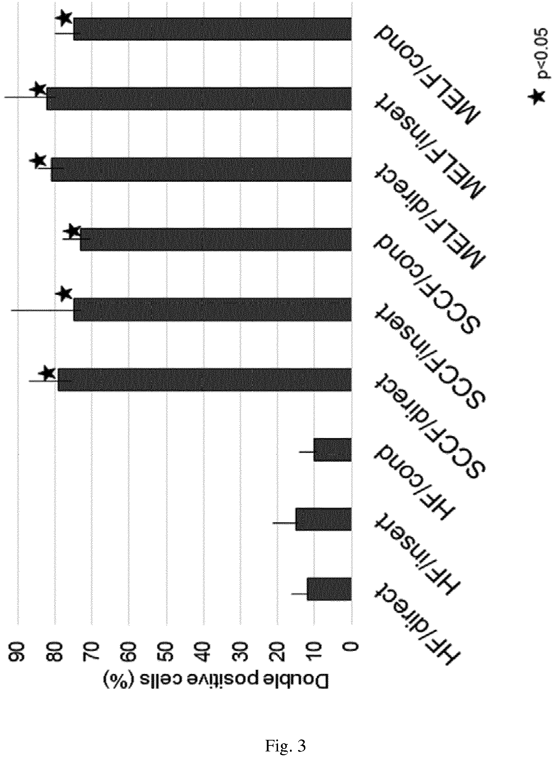

**Fig. 3****.** Effect of CAFs on K8+K14 co-expression in breast cancer cells: Cancer-associated fibroblasts SCCF and MELF significantly stimulate in contrary to normal dermal fibroblasts (HF) co-expression of both types of keratins (K8+K14) in directly co-cultured breast cancer cells EM-G3 as well as in EM-G3 separated by microporous membrane and in case where conditioned media are employed.

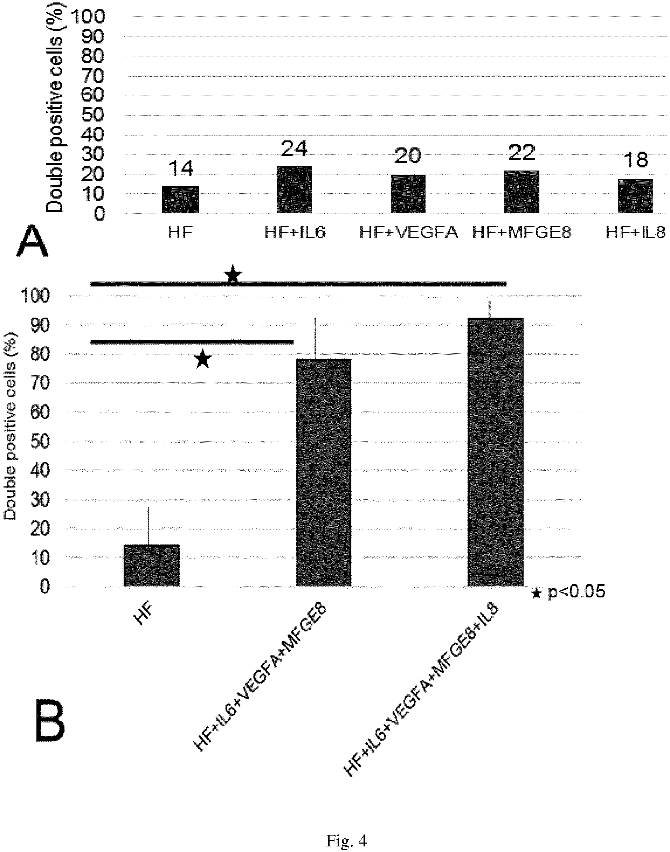

**Fig. 4****.** Effect of IL-6, VEGF-A, MFGE-8 and IL-8 on co-expression of keratin 8 and 14 in breast cancer cells: IL-6, VEGF-A, MFGE-8 and IL-8 stimulate co-expression of K8 and K14 in EM-G3 breast cancer cells cocultured with normal dermal fibroblasts (A). The simultaneous application of mentioned proteins has synergistic effect (B).

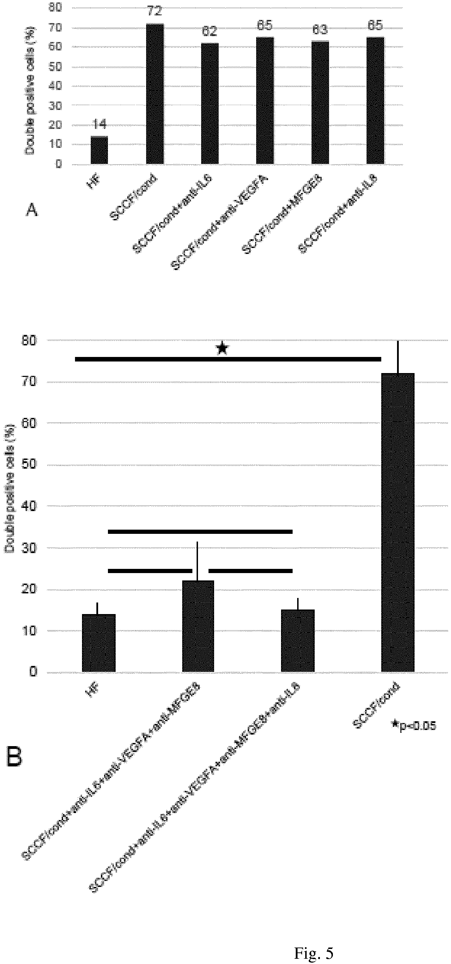

**Fig. 5****.** Effect of blocking antibodies on co-expression of keratin 8 and 14 in breast cancer cells: Introduction of antibodies against IL-6, VEGF-A, MFGE-8 and IL-8 to culture where EM-G3 were cultured with conditioned media from SCCF has small inhibitory effect on co-expression K8 and K14 in EM-G3 (A). This effect was strongly improved by simultaneous inhibition of all mentioned proteins (B).

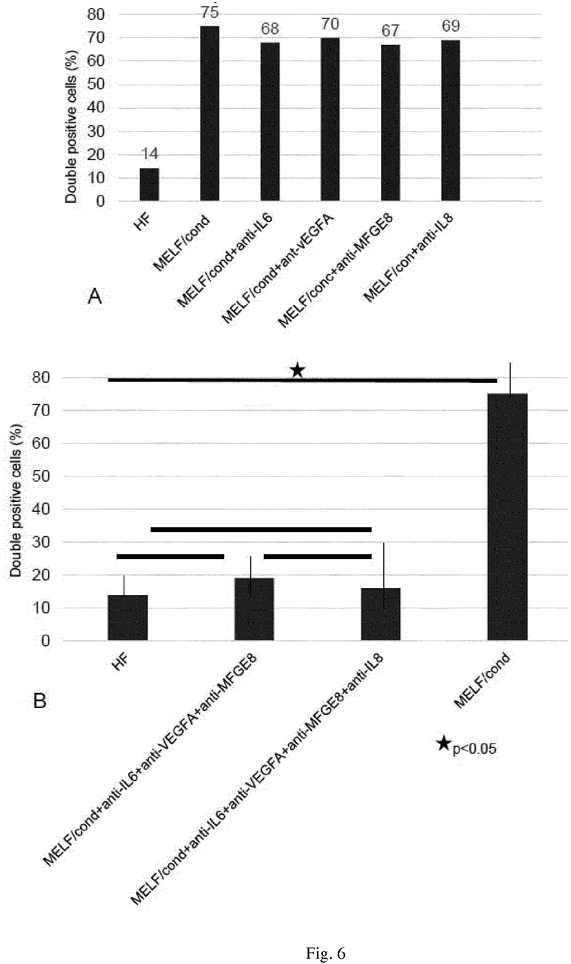

**Fig. 6****.** Effect of blocking antibodies on co-expression of keratin 8 and 14 in breast cancer cells: Introduction of antibodies against IL-6, VEGF-A, MFGE-8 and IL-8 to culture where EM-G3 were cultured with conditioned media from MELF has small inhibitory effect on co-expression K8 and K14 in EM-G3 (A). This effect was strongly improved by simultaneous inhibition of all mentioned proteins (B).

### Examples

### Materials and methods

The research was performed in promocultures of normal human fibroblasts (HF) and cancer-associated fibroblasts prepared form human basal cell carcinoma (BCCF), squamous cell carcinoma of tonsil (SCCF), skin metastase of breast cancer (BCMF) and skin metastase of melanoma (MELF). The breast cancer cell line EM-G3 (Dvořánková, B., Szabo, P., Lacina, L., Kodet, O., Matoušková, E., Smetana, K., Jr.: Fibroblasts prepared from different types of malignant tumors stimulate expression of luminal marker keratin 8 in the EM-G3 breast cancer cell line. Histochem. Cell Biol. 137: 679-685, 2012) was used for monitoring of coexpressio of keratin type 8 and 14 under the influence of studied fibroblasts and substances. Coexpression of both keratin type 8 and 14 indicates stem cell properties of cancer cells and so the highly unfavourable prognosis of patient (El-Rehim DMA, Pinder SE, Paish CE, Bell J, Blamey RW, Robertson JFR, Nicholson RI, Ellis IO: Expression of luminal and basal cytokeratins in human breast carcinoma. J Pathol. 203:661-671, 2004; Laakso M, Tanner M, Nilsson J, Wiklund T, Erikstein B, Kellokumpu Lehtinen P, Malmstro P, Wilking N, Bergh J, Isola J: Basoluminal carcinoma: a new biologically and prognostically distinct entity between basal and luminal breast cancer. Clin Cancer Res 12:4185-4191, 2006).

### Example 1: Characterization of CAFs by protein expression and enzymatic activity

CAFs from the squamous cell carcinoma of the head and neck (SCCF), skin metastasis of breast cancer (BCMF), basal cell carcinoma (BCCF), skin metastase of melanoma (MELF) and normal dermal fibroblasts were prepared and cultured as described (Lacina, L., Smetana, K., Jr., Dvořánková, B., Pytlik, R., Kideryová, L., Kučerová, L., Plzáková, Z., Štork, J., Gabius, H.-J., Andre, S.: Stromal fibroblasts from basal cell carcinoma affect phenotype of normal keratinocytes Brit. J. Dermatol. 156: 819-829, 2007). The cultured fibroblasts were characterized by measurements of their doubling time, expression of the proliferation marker Ki67, presence of α-smooth muscle actin, which is frequently present in CAFs, production of extracellular matrix molecules such as fibronectin and tenascin, and by enzymatic activity of senescence-associated β-galactosidase (potentially present in CAFs). Our results demonstrated heterogeneity of studied CAF types (Table 1).

**Table 1. Characterization of fibroblasts**

| Type of fibroblast | Doubling time (hours) | Ki67 positive cells (%) | α-SMA expression | Fibronectin expression | Tenascin expression | β-Gal activity |
|---|---|---|---|---|---|---|
| HF | 36 | < 10 | - | ++ | - | - |
| BCCF | 46 | < 10 | + | +++ | + | - |
| SCCF | 35 | < 10 | + | + | - | - |
| BCMF | 37 | < 10 | - | +++ | - | + |
| MELF | 57 | < 1 | + | + | - | +++ |

| | | | | | | |
|---|---|---|---|---|---|---|
| α-SMA: α-smooth muscle actin; β-Gal: senescence-associated β-galalactosidase. - negative; + positive; +++ strongly positive. | | | | | | |

### Example 2: CAFs differ from normal human fibroblasts in expression of a number of genes

Total RNA was isolated from CAFs using RNeasy Micro Kit (Qiagen Inc., USA) and rewritten and amplified to cRNA by Illumina TotalPrep RNA amplification kit (Ambion Inc., USA). The cRNA was hybridized on Human HT12-v4 whole genome expression arrays (Illumina, USA) and analyzed using BeadArray Reader (Illumina, USA). Resulting data were processed using Bioconductor (Orchestrating high-throughput genomic analysis with Bioconductor. W. Huber, V.J. Carey, R. Gentleman, ..., M. Morgan Nature Methods, 2015:12, 115.) package limma (Ritchie ME, Phipson B, Wu D, Hu Y, Law CW, Shi W and Smyth GK (2015). "limma powers differential expression analyses for RNA-sequencing and microarray studies." Nucleic Acids Research, 43(7), pp. e47.) to obtain lists of differentially expressed genes. The genes significantly upregulated in CAFs (SCCF, BCMF, BCCF and MELF) in comparison to normal dermal fibroblasts were further characterized. It was demonstrated that CAFs are clearly different from normal dermal fibroblasts (HF). Only 19 genes are upregulated in all types of CAFs and not in normal dermal fibroblasts (Table 2, Fig. 1B). The gene coding for IL-8 was also upregulated in all CAFs (but BCMF), yet did not reach statistical significance (Table 2). Among these genes, we have selected IL-6, MFGE-8, VEGF-A and IL-8 proteins for further analyses.

**Table 2. Genes with significantly upregulated activity in all studied CAFs**

| **Gene symbol** | **Gene name** |
|---|---|
| ACTG2 | actin, γ 2, smooth muscle, enteric |
| IGFBP2 | insulin-like growth factor binding protein 2, 36kDa |
| IGFBP7 | insulin-like growth factor binding protein 7 |
| **IL-6** | **interleukin 6 (interferon, β 2)** |
| JAG1 | jagged 1 |
| **VEGFA** | **vascular endothelial growth factor A** |
| MGP | matrix Gla protein |
| TFG | TRK-fused gene |
| PRICKLE 1 | prickle homolog 1 (Drosophila) |
| SFRP4 | secreted frizzled-related protein 4 |
| MSC | musculin |
| SVEP1 | sushi, von Willebrand factor type A, EGF and pentraxin domain containing 1 |
| SPRY1 | sprouty homolog 1, antagonist of FGF signaling (Drosophila) |
| **MFGE8** | **milk fat globule-EGF factor 8 protein (lactadherin)** |
| CRLF1 | cytokine receptor-like factor 1 |
| TPD52L1 | tumor protein D52-like 1 |
| CXCL16 | chemokine (C-X-C motif) ligand 16 |
| LTBP3 | latent transforming growth factor binding protein 3 |
| **IL8**^{(*)} | **chemokine (C-X-C motif) ligand 8** |

| | |
|---|---|
| ^{(*)}Upregulated in all CAFs except BCMF | |

### Example 3: Synergistic effect of IL6, IL8, VEGFA and MFGE8

Using STRING freely accessible bioinformatic system (https://string-db.org/, Szklarczyk D, Morris JH, Cook H, Kuhn M, Wyder S, Simonovic M, Santos A, Doncheva NT, Roth A, Bork P, Jensen LJ, von Mering C. The STRING database in 2017: quality-controlled protein-protein association networks, made broadly accessible. Nucleic Acids Res. 2017 Jan; 45:D362-68) we analyzed interaction of IL6, IL8, VEGFA and MFGE8 and the results obtained demonstrated that IL-6, IL-8 and VEGFA interact with similar groups of genes, which include genes that are very important for cancer growth and vascularization such as EGF, HGF, CXCL1, HIF1, TP53, TGFB1, JAK1/2, STAT2, STAT3 and VEGF-B/C (Fig. 2). On the other hand, MFGE-8 is responsible for interaction with another pathway, which includes integrins ITGAV, ITGB3 and ITGB5 (Fig. 2). Using the same bioinformatic tool we also demonstrated that targeting of IL-6, IL-8, VEGF-A and MFGE-8 influence the cluster of genes responsible A) for inflammation and cancer cell low differentiation status as well as their invasiveness B) vascularisation of tumors and C) resistence to hypoxia (Table 3). By inhibition of the combination of proteins IL6, IL8, VEGFA and MFGE8, the cancer growth, metastazation, vascularization and resistence to hypoxia can be minimized because the complex character of the block of these important genes minimizes the risk of "walk around" of the important genetic pathways (thus minimizing the induction of therapeutic resistence). Because resistence to hypoxia negatively influences therapeutic effort (Terry S, Buart S, Chouaib S.: Hypoxic stress-induced tumor and immune plasticity, suppression, and impact on tumor heterogeneity. Front Immunol. 8: 1625, 2017), its therapeutic maniplation can positively influence the efectivity of classical oncological therapy.

**Table 3. Cluster of genes/proteins influenced by inhibition of IL-6, IL-8, VEGF-A and MFGE-8**

| **Cluster of genes/proteins** | **Genes/proteins** |
|---|---|
| Inflammation, low differentiation status of cancer cells, invasiveness | IL-6, IL-6R, IL-6ST, IL-8, CXCR-1, CXCR-2, JAK-1, JAK-2, STAT-3, CCL-5, SOCS-3, CXCL-1, IL-17A, IL-17RA, LIF, MFGE-8, |
| Cancer vascularisation | PGF, NRP-1, FIGF, VEGF-A, VEGF-B, CDH-5, FLT-4, KDR-5, EGF, SRC |
| Resistance to hypoxia | ARNT, HIF-1A, HIF-1AN, CREBBP, EGLN-1, EGLN-3, VHL, FLT-1, |

*Blocking of the proteins by neutralizing antibodies reverts the phenotype*
For analysis of influence of CAFs on cancer cells, we selected normal dermal fibroblasts (HF) as a control, ectoderm-originated SCCF and neuroectoderm-originated MELF as CAFs and breast cancer cells EM-G3 as reporter cancer cells. All types of fibroblasts and breast cancer cells were cultured as described in (Dvořánková, B., Szabo, P., Lacina, L., Kodet, O., Matoušková, E., Smetana, K., Jr.: Fibroblasts prepared from different types of malignant tumors stimulate expression of luminal marker keratin 8 in the EM-G3 breast cancer cell line. Histochem. Cell Biol. 137: 679-685, 2012). Specifically, we monitored co-expression of keratins type 8 and 14. Their simultaneous expression is typical in breast cancer cells with aggressive phenotype (El-Rehim DMA, Pinder SE, Paish CE, Bell J, Blamey RW, Robertson JFR, Nicholson RI, Ellis IO Expression of luminal and basal cytokeratins in human breast carcinoma. J Pathol 203:661-671, 2004; Laakso M, Tanner M, Nilsson J, Wiklund T, Erikstein B, Kellokumpu-Lehtinen P, Malmstro P, Wilking N, Bergh J, Isola J: Basoluminal carcinoma: a new biologically and prognostically distinct entity between basal and luminal breast cancer. Clin Cancer Res 12:4185-4191, 2006). The cells were co-cultured in direct co-culture, using a transwell system, where both cell populations were separated by a microporous membrane (pore diameter 0.2 µm) and in culture, where conditioned medium from cultured CAFs was supplemented to EM-G3 culture.

*Results of co-culture experiments:* CAFs prepared from squamous cell cancer (SCCF) as well as from skin metastasis of malignant melanoma (MELF) significantly stimulated co-expression of keratin type 8 and 14 in both direct and transwell regimen. Conditioned medium from the CAFs had similar stimulatory effect. Statistical significance was evaluated using Student's paired t-test (Fig. 3).

*Results of supplementation of culture medium with IL-6, IL-8, VEGF-A and MFGE-8:* Culture of normal dermal fibroblasts (HF) supplemented with one of the IL-6, VEGFA-A, IL-8 and MFGE-8 stimulated coexpression of keratin type 8 and keratin type 14 in EM-G3 in comparison with normal dermal fibroblasts (Fig. 4A), however much higher and more significant increase was observed when the culture of normal dermal fibroblasts was supplemented with the combination of IL-6, VEGFA-A, and MFGE-8 (Fig. 4B). Addition of IL-8 further increased number of double positive cells. Statistical significance was evaluated using Student's paired t-test.

*Results of supplementation of conditioned media from CAFs with monoclonal antibodies blocking activity of IL-6, VEGF-A, MFGE-8 and IL-8:* EM-G3 cultured in presence of conditioned media from culture of SCCF (Fig. 5) and MELF (Fig. 6) were treated by 10-30 µg/ml (w/v) of one of anti- IL-6, VEGF-A, MFGE-8 and IL-8 antibodies, respectively (Figs. 5A and 6A). The results demonstrated a slight (about 10%) reduction of number of EM-G3 cells double positive for keratin type 8 and 14. In a further study, EM-G3 cultured in presence of conditioned media from culture of SCCF and MELF were treated by 10-30 µg/ml (w/v) of the combination of anti- IL-6, VEGF-A, and MFGE-8 antibodies, with and without the same concentration of antibody blocking IL-8 activity (Figs. 5B and 6B). The results clearly demonstrated that antibodies against IL-6, VEGF-A and MFGE-8 very significantly reduced number of EM-G3 cells double positive for keratin type 8 and 14. Addition of antibody against IL-8 further improved the inhibitory effect. Statistical significance was evaluated using Student's paired t-test.

## Claims

1. A combination of at least one IL-6 inhibitor, at least one VEGF-A inhibitor, and at least one MFGE-8 inhibitor.

2. The combination according to claim 1, **wherein**
- IL-6 inhibitor is selected from a low-molecular inhibitor of the IL-6 protein, a low-molecular inhibitor of the IL-6 receptor, an anti-IL-6 antibody, an anti-IL-6R antibody, a Fab fragment of anti-IL-6 antibody, a Fab fragment of anti-IL-6R antibody;
- the VEGF-A inhibitor is selected from a low-molecular inhibitor of the VEGF-A protein, a low-molecular inhibitor of the VEGF-A receptor, an anti- VEGF-A antibody, an anti- VEGF-AR antibody, a Fab fragment of anti- VEGF-A antibody, a Fab fragment of anti- VEGF-AR antibody;
- MFGE-8 inhibitor is selected from a low-molecular inhibitor of the MFGE-8 protein, a low-molecular inhibitor of the MFGE-8 receptor, an anti- MFGE-8 antibody, an anti- MFGE-8R antibody, a Fab fragment of anti- MFGE-8 antibody, a Fab fragment of anti- MFGE-8R antibody.

3. The combination according to claim 1 or 2, **characterized in that** the combination contains anti- IL-6, anti-VEGF-A and anti-MFGE-8 antibodies and/or Fab fragments thereof.

4. The combination according to any one of the preceding claims, **characterized in that** it further comprises at least one IL-8 inhibitor.

5. The combination according to claim 4, **characterized in that** the at least one IL-8 inhibitor is selected from a low-molecular inhibitor of the IL-8 protein, preferably reparixin, a low-molecular inhibitor of the IL-8 receptor, an anti-IL-8 antibody, an anti-IL-8R antibody, a Fab fragment of anti-IL-8 antibody, a Fab fragment of anti-IL-8R antibody.

6. The combination according to any one of the preceding claims, **characterized in that** the antibodies or Fab fragments thereof are humanized.

7. The combination according to any one of the preceding claims, **characterized in that** the inhibitors are present in molar ratios IL-6 inhibitor : VEGF-A inhibitor : MFGE-8 inhibitor 1-5 : 1-5 : 1-5.

8. The combination according to claim 4, **characterized in that** the inhibitors are present in molar ratios IL-6 inhibitor : VEGF-A inhibitor : MFGE-8 inhibitor : IL-8 inhibitor 1-5 : 1-5 : 1-5 : 1-5.

9. A pharmaceutical preparation, **characterized in that** it contains the combination of inhibitors according to any one of the preceding claims and at least one pharmaceutically acceptable carrier.

10. The pharmaceutical preparation according to claim 9, **characterized in that** it further contains at least one cytostatic.

11. The pharmaceutical preparation according to claim 9 or 10, **characterized in that** the concentrations of anti- IL-6 antibody, anti-IL-6R antibody, the Fab fragment of anti-IL-6 antibody, the Fab fragment of anti-IL-6R antibody, anti-VEGF-A antibody, anti- VEGF-AR antibody, a Fab fragment of anti- VEGF-A antibody, a Fab fragment of anti- VEGF-AR antibody, anti-MFGE-8 antibody, anti- MFGE-8R antibody, a Fab fragment of anti- MFGE-8 antibody, a Fab fragment of anti- MFGE-8R antibody, anti-IL-8 antibody, an anti-IL-8R antibody, a Fab fragment of anti-IL-8 antibody, a Fab fragment of anti-IL-8R antibody are in concentrations ranging from 10 to 50 µg/ml.

12. The combination according to any one of the claims 1 to 8 and/or the pharmaceutical preparation according to any one of the claims 9 to 11 for use as a medicament.

13. The combination according to any one of the claims 1 to 8 and/or the pharmaceutical preparation according to any one of the claims 9 to 11 for use in the treatment of cancer.

14. The combination and/or the pharmaceutical preparation for use according to claim 13, **characterized in that** the cancer is selected from the group comprising breast cancer, malignant melanoma, head and neck squamous cell carcinoma.
